# EUROPEAN PATENT SPECIFICATION

(11) **EP 2 034 967 B1**
(45) Date of publication and mention of the grant of the patent: **18.05.2011**
(21) Application number: 07730181.0
(22) Date of filing: 15.06.2007
(51) Int. Cl.: A61K 9/20, A61K 9/48, A61K 31/55, A61K 45/08

(54) **ORAL PHARMACEUTICAL COMPOSITION OF A POORLY WATER-SOLUBLE ACTIVE SUBSTANCE**
ORALE PHARMAZEUTISCHE ZUSAMMENSETZUNG EINER SCHWER WASSERLÖSLICHEN WIRKSUBSTANZ
COMPOSITION PHARMACEUTIQUE ORALE D'UNE SUBSTANCE ACTIVE PEU SOLUBLE DANS L'EAU

(30) Priority: 16.06.2006 US 814076 P; 16.06.2006 EP 06115582
(43) Date of publication of application: 18.03.2009
(73) Proprietor: Abbott Healthcare Products B.V., 1381 CP Weesp (NL)
(72) Inventor: GORISSEN, Henricus R.M., NL-1381 CP Weesp (NL)
(74) Representative: Verhage, Marinus
(86) International application number: PCT/EP2007/055937
(87) International publication number: WO 2007/144418

(56) References cited:
- WO-A-03/068266
- WO-A-2004/062692
- WO-A-2006/067150
- WO-A-2007/054975

## Description

The present invention relates to an improved oral formulation of an active compound of the general formula 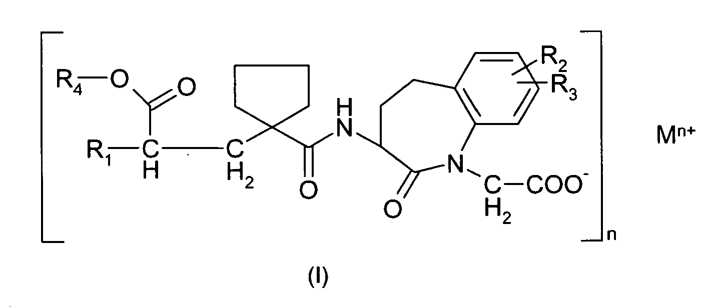 wherein:
R₁ is chosen from:
   (1) (C₁-C₆)alkoxy(C₁-C₆)alkyl, which is optionally substituted by a (C₁-C₆)alkoxy;
   (2) phenyl-(C₁-C₆)-alkyl and phenyloxy-(C₁-C₆)-alkyl, wherein the phenyl group is optionally substituted with (C₁-C₆)alkyl, (C₁-C₆)alkoxy or halogen; and
   (3) naphtyl-(C₁-C₆)-alkyl;
R₂ and R₃ are both independently hydrogen or halogen,
R₄ is a biolabile ester forming group,
M is a hydrogen or a metal ion, preferably a bivalent metal ion, and
nis1,2 or 3.

These compounds and their salts and biolabile esters fall under the scope of protection of the present invention and are potent ECE/NEP inhibitors and are described in Waldeck et al., US 5,677,297 and EP 0733642. The benzazepine-N-acetic acid compounds used in the present invention are known from EP 0733642, EP 0830863, WO 00/48601 and WO 01/03699, and can be produced by the methods described in said US 5,677,297 and EP 0733642. These patents are related to said compounds and their physiologically acceptable salts as such and to the use of the compound in heart insufficiency. WO 03/059939 relates to specific salts of these compounds, especially to the calcium salt. EP 0830863, WO00/48601 and WO01/03699 are related to the use of the above compounds in the improvement of gastrointestinal blood flow, in the treatment of hypertension and in the treatment and prophylaxis of cardiac damages induced by adriamycin and comparable anti-cancer drugs, respectively.

Various active compounds, including the compounds of formula (I) mentioned above have a very poor solubility in water. When these active compounds are administered to the body, they often have a poor bio-availability due to the poor solubility in the digestive fluid. In order to solve this problem several methods were developed, such as micronization, inclusion in cyclodextrins, the use of inert watersoluble carriers, the use of solid dispersions (WO 00/00179) or solid solutions or nanocrystalline or amorphous forms of the active compound.

WO 03/068266 describes an oral solid solution formulation of compounds of formula (I) having enhanced bio-availability compared with said active compound in a traditionally formulated form. Although this formulation has good bioavailability properties, it has the draw-back that it is formed via a melt mixture leading to some restrictions: it has to be formulated either into a capsule, or into a tablet via melt-extrusion technique. Further the size of the formulation will be too large for higher dosages.

WO 2006/067150 (not pre-published) describes an oral immediate release formulation of compounds of formula (I) comprising the active compound in an amount up to 60% of the total weight of the formulation, at least 10 % w/w of an alkaline compound or a mixture of alkaline compounds, between 0.1 and 10% w/w of one ore more surfactants and optionally auxiliary materials in an amount of between 1% and 45% of the total weight of the formulation. Especially when docusate sodium is used as the surfactant a good bioavailability of the active compound is obtained.

It is the objective of the present invention to provide an alternative oral formulation for the compound of formula I as defined above with a significant increase in bio-availability compared with said active compound in a traditionally formulated form that is sufficiently stable for commercial use and that also can be used to prepare formulations with a high content of active compound with a reasonable size. It is a further objective of the present invention to provide a formulation that can be prepared using normal formulation procedures and equipment, so that no large investments are necessary.

This objective can be achieved, according to the present invention, by an improved oral formulation of an active compound of the general formula 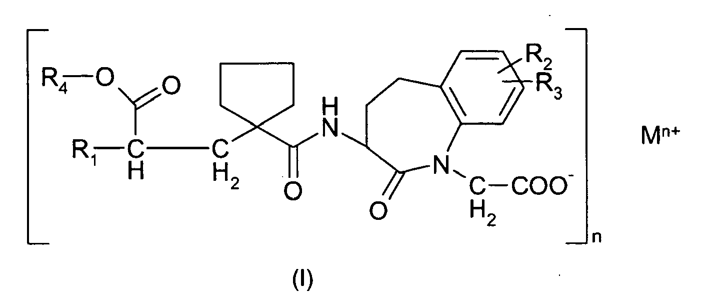 wherein:
R₁ is a selected from the group consisting of (C₁-C₆)alkoxy(C₁-C₆)alkyl which may be substituted by a (C₁-C₆)alkoxy, phenyl-(C₁-C₆)-alkyl and phenyloxy-(C₁-C₆)-alkyl wherein the phenylgroup may be substituted with (C₁-C₆)alkyl, (C₁-C₆)alkoxy or halogen, and naphtyl-(C₁-C₆)-alkyl,
R₂ and R₃ are both independently hydrogen or halogen,
R₄ is a biolabile ester forming group,
M is a hydrogen or a metal ion, preferably a bivalent metal ion.
n is 1, 2 or 3;
   comprising
   a) said active compound in an amount of between 10% and 80 % of the total weight of the formulation;
   b) at least 10% w/w an alkaline compound or a mixture of alkaline compounds;
   c) optionally comprises auxiliary materials in an amount of between 1% and 45% of the total weight of the formulation
      with the proviso that the formulation does not contain a surfactant.

M is preferably selected from the group consisting of Li+, Ca²⁺, Mg²⁺ and Zn²⁺, and is most preferably Ca²⁺. (C₁-C₆)-alkyl is defined as a straight or branched alkyl group consisting of between 1 and 6 carbon atoms. (C₁-C₆)-alkoxy is defined as a straight or branched alkoxy group consisting of between 1 and 6 carbon atoms. R₁ is preferably phenylethyl, R₂ and R₃ are preferably hydrogen and R₄ is preferably ethyl.

In the framework of the present invention suitable R₄ groups that can form biolabile esters include lower alkyl groups, phenyl or phenyl-lower-alkyl groups which are optionally substituted in the phenyl ring by lower alkyl or by a lower alkylene chain bonded to two adjacent carbon atoms, dioxolanylmethyl groups which are optionally substituted in the dioxolane ring by lower alkyl, or C2 -C6-alkanoyloxymethyl groups which are optionally substituted on the oxymethyl group by lower alkyl. Where the group R₄ forming a biolabile ester is lower alkyl, this can be a preferably unbranched alkyl group with 1 to 4, preferably 2, carbon atoms. Where the group forming a biolabile ester is an optionally substituted phenyl-lower-alkyl group, its alkylene chain can contain 1 to 3, preferably 1 carbon atoms. Where the phenyl ring is substituted by a lower alkylene chain, this can contain 3 to 4, in particular 3, carbon atoms. Particularly suitable phenyl-containing substituents R₄ are phenyl, benzyl or indanyl. Where R₄ is an optionally substituted alkanoyloxymethyl group, its alkanoyloxy group can contain 2 to 6, preferably 3 to 5, carbon atoms and is preferably branched and can be, for example, a pivaloyloxymethyl radical (tert-butylcarbonyloxymethyl radical).

The preferred compound is the calcium salt of acid, 3-[[[1-[2-(ethoxycarbonyl)-4-phenylbutyl]cyclopentyl]carbonyl]amino]-2,3,4,5-tetrahydro-2-oxo-1 H-1-benzazepine-1-acetic. The most preferred compound is said compound in its 3S,2'R form, also known as daglutril calcium or SLV 306 Calcium. This compound is referred to as Compound S-Ca, the corresponding acid (3-[[[1-[2-(ethoxycarbonyl)-4-phenylbutyl]-cyclopentyl]-carbonyl]amino]-2,3,4,5-tetrahydro-2-oxo-1 H-1-Benzazepine-1-acetic acid), also known as daglutril or SLV306 is referred to as Compound S-H.

The active compound of formula (I) is normally used in an amount between about 10 and 80% by weight, more preferably in an amount between 15 and 75% by weight, even more preferably in an amount between 20 and 65% by weight and most preferably in an amount between about 45 and 65% by weight. The active compound is or may optionally be used in a micronized form.

The following definitions are provided to facilitate understanding of certain terms used within the framework of the present application.
Sufficiently stable for commercial use means an acceptable chemical and physical stability during a storage period of at least one year at ambient conditions, preferably at least 2 years, even more preferably at least 3 years and most preferred at least 5 years. An acceptable chemical stability means not more than 5% degradation of the active material during the storage period, preferably not more than 3% and most preferably not more than 1%. An acceptable physical stability means no significant change in appearance, no breaking of tablet during deblistering at the end of the storage period and not more than 20% change of the disintegration time. The term "micronized" refers to a particle size wherein, on a volume basis, more than 95% of the particles is smaller than 75 microns.
Surfactants are defined as molecules with well defined polar and non-polar regions that allow them to aggregate in solutions to form micelles. Depending on the nature of the polar area, surfactants can be non-ionic, anionic, cationic and zwitterionic. Examples of non-ionic hydrophilic surfactants are polyoxyethylene sorbitan esters, cremophores and poloxamers. Examples of anionic surfactants are sodium lauryl sarcosinate, docusate and pharmaceutically acceptable docusate salts such as docusate calcium, docusate sodium and docusate potassium.

The alkaline compound is selected from the group consisting of inorganic and organic alkaline compounds, such as sodium bicarbonate, potassium bicarbonate, sodium carbonate, potassium carbonate, sodium citrate, tris buffer, triethanolamine, alkaline hydroxides such as sodium hydroxide, potassium hydroxide or magnesium hydroxide, alkaline phosphates such as dipotassium hydrogen phosphate, and meglumine. Also mixtures of these alkaline compounds can be used. Preferred alkaline compounds are sodium bicarbonate, potassium bicarbonate, sodium carbonate, potassium carbonate and calcium carbonate. An even more preferred alkaline compound is sodium carbonate.

Preferably the alkali system is present in an amount greater than 10% w/w of the uncoated composition, more preferably greater than 20% w/w, or is present in an amount greater than 25%, 30% w/w, 40% w/w, 45% w/w, 50% w/w, 55% w/w or 60% w/w of the uncoated composition. The term "uncoated formulation" means a formulation before the application of the optional coating material(s).
In case a carbonate is used, it is preferably used in an amount of 25% of the total weight of the uncoated formulation or it is present in an amount of at least 45% w/w, or is present in an amount greater than 50% w/w, 55% w/w or 60% w/w of the uncoated formulation.

In a further preferred embodiment the invention relates to an improved oral pharmaceutical composition of an active compound of the general formula 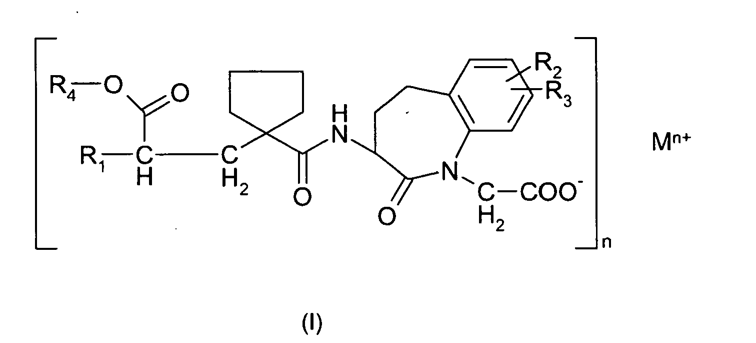 wherein:
R₁ is a selected from the group consisting of (C₁-C₆)alkoxy(C₁-C₆)alkyl which may be substituted by a (C₁-C₆)alkoxy, phenyl-(C₁-C₆)-alkyl and phenyloxy-(C₁-C₆)-alkyl wherein the phenylgroup may be substituted with (C₁-C₆)alkyl, (C₁-C₆)alkoxy or halogen, and naphtyl-(C₁-C₆)-alkyl,
R₂ and R₃ are both independently hydrogen or halogen,
R₄ is a biolabile ester forming group,
M is a hydrogen or a metal ion, preferably a bivalent metal ion.
n is 1, 2 or 3;
   comprising
   a) said active compound in an amount of 10 to 65% of the total weight of the formulation;
   b) at least 10% w/w of a sodium carbonate having a particle size distribution wherein more than 97% of the particles are smaller than 500 µm, more than 40% of the particles are smaller than 160 µm and more than 10% of the particles are smaller than 63 µm;
   d) optionally comprises auxiliary materials in an amount of between 1% and 45% of the total weight of the formulation;

In this preferred embodiment, the alkali system comprises a sodium carbonate having a smaller particle size than normal sodium carbonate, which has a particle size distribution (determined by sieve analysis and based on weight) wherein at most 25% of the particles are smaller than 160 µm. This sodium carbonate has also higher specific surface area than normal sodium carbonate, which has a specific surface area (determined according to the standard BET area measurement) of approximately 0.2 m²/g. As indicated above, the sodium carbonate used in the preferred embodiment has a particle size distribution (determined by sieve analysis and based on weight) wherein more than 97% of the particles are smaller than 500 µm, more than 40% of the particles are smaller than 160 µm and more than 10% of the particles are smaller than 63 µm. In an even more preferred embodiment more than 98% of the particles are smaller than 500 µm, more than 60% of the particles are smaller than 160 µm and more than 30% of the particles are smaller than 63 µm. The specific surface area is preferably higher than 1 m²/g, and more preferably higher than 1.5 m²/g. The most preferred sodium carbonate is the special sodium carbonate marketed by Solvay SA as Soda Ash IPH. In this type of sodium carbonate typically 99.8 % of the particles is smaller than 500 µm, 80% of the particles is smaller than 160 µm and 40% of the particles is smaller than 63 µm and this type of sodium carbonate has a specific surface area of 2 m²/g.

Surprisingly the inventors of the present invention have found that using an alkaline compound in the formulation, alone or in a mixture, even without any surfactant in the composition, prevents the formation of a difficult to solubilize gel in the acid gastric fluid, thereby enhancing the solubility of SLV-306 particularly as evidenced during in vitro dissolution studies in a biphasic dissolution model (see Example 1a), which indicates an improvement in the in vivo solubility as well as well as an improvement in bioavailability. Especially when using sodium carbonate with a mean particle size of 100 µm and a specific surface area of 2 m²/g such as (Soda Ash IPH) a formulation with a good *in vivo* solubility and a good bioavailability is obtained. Further the compositions are expected to have a good stability upon storage.

In case the abovementioned carbonate with specific mean particle size and surface area is used, it is preferably used in an amount of at least 15% of the total weight of the uncoated formulation, more preferably in an amount of at least 18%, even more preferably in an amount of at least 20%, or it is present in an amount greater than 25% w/w, 30% w/w, 40% w/w, 50% w/w or 60% w/w of the uncoated formulation.

Specific solid alkaline compounds like the bicarbonates and carbonates as indicated above are often used in combination with solid acidic compounds (e.g. citric acid, tartaric acid, adipic acid, fumaric acid, succinic acid, ascorbic acid, nicotinic acid, saccharin, aspirin, malic acid, sodium dihydrogen phosphate, disodium dihydrogen pyrophosphate, sodium dihydrogen citrate and disodium hydrogen citrate) in effervescent compositions. In the present invention the composition preferably does not contain an acidic compound.

The formulation optionally comprises auxiliary materials at an amount of up to 45% of the total weight of the formulation and preferably between 1% and 45% of the total weight of the formulation. Examples of these auxiliary materials include, but are not limited to:
a) Binders including, but not limited to acacia, alginic acid and salts thereof, cellulose derivatives, methylcellulose, hydroxyethyl cellulose, hydroxypropyl cellulose, polyethylene glycol, gums, polysaccharide acids, hydroxypropyl methylcellulose, gelatin, polyvinylpyrrolidone, polyvinylpyrrolidone/vinyl acetate copolymer, polymethacrylates, hydroxypropylmethylcellulose, starch, pregelatinized starch, ethylcellulose, tragacanth, dextrin, microcrystalline cellulose, sucrose, or glucose, and the like.
b) Disintegration agents including, but not limited to starches, pregelatinized corn starch, pregelatinized starch, celluloses, cross-linked carboxymethylcellulose, crospovidone, cross-linked polyvinylpyrrolidone, a calcium or a sodium alginate complex, clays, alginates, gums, or sodium starch glycolate, and any disintegration agents used in tablet preparations.
c) Filling agents including, but not limited to lactose, calcium carbonate, calcium phosphate, dibasic calcium phosphate, calcium sulfate, microcrystalline cellulose, cellulose powder, dextrose, dextrates, dextran, starches, pregelatinized starch, sucrose, xylitol, lactitol, mannitol, sorbitol and the like.
d) Stabilizers including, but not limited to any antioxidation agents, buffers, or acids, and the like.
e) Lubricants including, but not limited to magnesium stearate, calcium hydroxide, talc, colloidal silicon dioxide, sodium stearyl fumarate, hydrogenated vegetable oil, stearic acid, glyceryl behenate, magnesium, calcium and sodium stearates, waxes, Stearowet, boric acid, sodium benzoate, sodium acetate, DL-leucine, polyethylene glycols, sodium oleate, or sodium lauryl sulfate, and the like.
f) Wetting agents including, but not limited to oleic acid, glyceryl monostearate, sorbitan monooleate, sorbitan monolaurate, triethanolamine oleate, polyoxyethylene sorbitan monooleate, polyoxyethylene sorbitan monolaurate, sodium oleate, or sodium lauryl sulfate, and the like.
g) Diluents such lactose, starch, mannitol, sorbitol, dextrose, microcrystalline cellulose, dibasic calcium phosphate, sucrose-based diluents, confectioner's sugar, monobasic calcium sulfate monohydrate, calcium sulfate dihydrate, calcium lactate trihydrate, dextrates, inositol, hydrolyzed cereal solids, amylose, powdered cellulose, calcium carbonate, glycine, or bentonite, and the like.
h) Anti-adherents or glidants including, but not limited to colloidal silica, talc, corn starch, DL-leucine, sodium lauryl sulfate, and magnesium, calcium, or sodium stearates, and the like.
i) Pharmaceutically compatible carriers including, but not limited to acacia, gelatin, colloidal silicon dioxide, calcium glycerophosphate, calcium lactate, maltodextrin, glycerine, magnesium silicate, sodium caseinate, soy lecithin, sodium chloride, tricalcium phosphate, dipotassium phosphate, sodium stearoyl lactylate, carrageenan, monoglyceride, diglyceride, or pregelatinized starch, and the like.
In the case of the preferred embodiment, wherein the formulation contains at least 10% w/w of the sodium carbonate having a specific particle size distribution and/or surface area as described above, the formulation may also contain a surfactant as auxiliary material.

The final formulation is preferably in the form of granules, compressed tablets, or capsules.

The formulation described above can be prepared using conventional formulation procedures and equipment. Therefore it is another aspect of the present invention to provide a method of preparing a formulation as described above comprising the following steps:
a) mixing of the active compound of formula I with an alkaline compound or a mixture of alkaline compounds and optionally with one or more of the auxiliary materials;
b) compacting the mixture
c) milling and sieving of the granules obtained from said compacting and optionally mixing said sieved granules with one or more auxiliary materials; and
d) optionally compressing of the mixture into tablets, optionally followed by coating, and/or optionally filling the mixture into capsules.

In another embodiment of the invention, the formulation is prepared with an organic granulation method comprising the following steps:
a) mixing of the active compound with the one or more auxiliary materials;
b) granulating said mixture with an organic solvent;
c) removing the organic solvent to obtain granules;
d) milling and sieving the granules and mixing of the sieved granules with the remaining portion of the auxiliary materials;
e) optionally compressing the mixture into tablets, optionally followed by coating, and/or optionally filling the mixture into capsules.
In the organic granulation method several organic solvents can be used. Examples are methyl t-butyl ether (MTBE), dichloromethane and ethyl acetate. The preferred organic solvent to be used is ethyl acetate.

When the formulations of the present invention are provided in the form of tablets, these tablets have disintegration times of between 5 minutes and 90 minutes. Preferably the disintegration times are below 60 minutes and most preferably they are below 45 minutes. Formulations with short disintegration times can be prepared by using a porous sodium carbonate as available in Soda Ash IPH.

Various additional steps can also be part of the process, such as drying, breaking, sieving, mixing and packaging, but these steps are no essential features in obtaining the formulation according to the present invention.

In another aspect the invention provides a composition having a favorable release profile. Therefore the present invention is also related to oral pharmaceutical compositions as described above, wherein the composition has a dissolution of at least 50% within 5 minutes as measured using the USP apparatus 2 configuration at a paddle speed of 50 rpm at 37.0 °C and at pH 6.8. The dissolution after 5 minutes is preferably at least 55% and even more preferably 60%. After 15 minutes the dissolution is at least 65%, preferably at least 70% and more preferably 75%. After 30 minutes the dissolution is at least 75%, preferably at least 80% and more preferably at least 85%.

At a pH of 2.0 the pharmaceutical composition according to the present invention does not significantly release the active compound. Less than 5% of the active compound is released within 30 minutes as measured using the USP apparatus 2 configuration at a paddle speed of 50 rpm at 37.0 °C and at pH 2.0. Preferably less than 2% is released and most preferably less than 1 %.

The following examples are intended to further illustrate the invention, in more detail, and therefore these example are not deemed to restrict the scope of the invention in any way.

### EXAMPLES.

**Example 1. Materials, equipment and methods**

### Materials.

S-Ca was prepared according to the prescription given in Examples 2 and 3 of WO03/059939 starting with the acid prepared according to Example 2 of EP 0733642.
Soda Ash IPH (also indicated as Soda, porous or porous soda) were obtained from Solvay SA Brussels, Belgium.
All other auxiliary materials are readily commercially available.

### Equipment

For roller compaction a Fitzpatrick type IR200 roller compactor was used, equipped with a Fitzmill L1A.
The settings of the roller compaction were:
Rotational speed horizontal feeding screw (HFS in rpm)
Rotational speed vertical feeding screw (VFS in rpm)
Rotational speed rollers (N1 in rpm)
Gap (d in mm)
Compaction force (F in KN/cm)
The settings of the Fitzmill were:
Rotational speed hammer knifes (N2 in rpm)
Screen size (in mm)

Methods.

### a) Description of the bi-phase in-vitro dissolution method.

The bi-phase dissolution was performed with the USP apparatus 2 configuration. The paddle speed was 50 rpm and the temperature of the vessels (and so the dissolution medium) was maintained at 37.0 °C using Vankel VK7010 equipment.
The dissolution of the formulations was started in 500 ml 0.1 M hydrochloric acid (4.2 ml concentrated hydrochloric acid (HCl) in 500 ml water)(phase 1). After 0, 5, 15, and 30 minutes a sample was taken. After 30 minutes 500 ml 1M phosphate buffer (32.4 gram sodium di-hydrogen phosphate NaH₂PO₄ and 124.8 gram di-sodium hydrogen phosphate (Na₂HPO₄) in 1000 ml water was added to phase 1. Addition of the phosphate buffer changed the pH of the dissolution medium from pH 1 in phase 1 to pH 6.8 in phase 2. During the dissolution test the pH of both phases remained unchanged. Samples were taken after 35, 45, and 60 minutes.
All the samples were filtered through a Pall Zymark Acrodisc PSF, GxF/GHP, 0.45 µm or a Millipore Millex-FH (hydrophobic PTFE 0.45 µm) filter.
The quantity of the dissolved daglutril in the filtered samples was analyzed by off-line UV measurements at 240 nm using external standardization.
In an earlier comparative study with the calcium salt of the compound SLV306 (S-Ca) it was shown that this bi-phase *in vitro* dissolution method had a good correlation with *in-vivo* results.

### b) description of methods to characterize porous sodium carbonate.

The particle size distribution of the porous sodium carbonate was measured by means of mechanical system. A sample of about 70 g of the product was weighed and placed into the upper sieve of a sifting machine (an automatic device which can transfer a combination of horizontal movements and vertical jerk movements to a set of sieves, e.g. a ROTAP or AS 200 RETSCH sifting machine) containing sieves with screens of 0.5, 0.25, 0.16, 0.125, 0.1, 0.063, and 0.045 mm. The sieving procedure was performed for about 15 minutes. The content of each sieve was weighed and the mass of the particles having a particle size less than 500 µm, the mass of the particles having a particle size less than 160 µm, and the mass of the particles having a particles size less than 63µm were calculated. The specific surface area of the porous sodium carbonate was measured according to the standard BET area method.

### c) description of other physical methods.

The powder flow was measured in a brass funnel with Ø 8 mm outlet. Powder flow was expressed in sec/100 gram.
- The particle size distribution of roller-compacted material was obtained from manual sifting using 0.25 mm, 0.50 mm, 0.71 mm, 0.85 mm 1.0 mm, and 2.0 mm screens. The amount (%) < 0.25 mm, the amount (%) > 1.0 mm and d(50%) were calculated. The bulk and tapped density of a mixed granulate was determined as follows:
   - An amount of 100 to 150 gram granulate was filled in a graduated cylinder.
   - The occupied volume was determined.
   - After 1200 taps, the occupied volume was determined again. The Carr-index was calculated from bulk and tapped volume.
Crushing strength of the tablets was determined by crushing five tablets in a Schleuniger hardness tester. The mean value was reported.
Friability was determined on 20 tablets using an Erweka friability tester. The test conditions were 10 minutes at 40 rpm. The friability was expressed in % tablet weight loss.
Disintegration was tested on one tablet, using water as the dissolution medium.

**Example 2: Preparation of a traditionally formulated coated tablet of S-Ca.**

| **Ingredients** | **Quantity (mg/tablet)** |
|---|---|
| | **Tablet 400/700mg** |
| S-Ca | 414.25 |
| Micro crystalline cellulose PH301 | 249.00 |
| Cross-linked polyvinylpyrrolidon | 14.00 |
| Sodium stearyl fumarate | 1.75 |
| Opadry II Yellow coating | 21.00 |
| Tablet mass | 700.00 |

**Procedure:**
i) S-Ca was compacted and passed through a 1.0 mm sieve.
ii) The material of step (i) was mixed with micro crystalline cellulose PH301, cross-linked polyvinylpyrrolidon and sodium stearyl fumarate to obtain a uniform mixture.
iii) The material of step (ii) was compressed using a tablet compression machine.
iv) The tablets from step (iii) were coated in suitable coating equipment.

### Example 3: Preparation of uncoated tablets of S-Ca containing Soda Ash IPH

### 1. Roller compaction of part of the ingredient

| Batch granulate obtained | A | B | C | D | E |
|---|---|---|---|---|---|
| S-Ca | 622 | 622 | 622 | 622 | 622 |
| Soda, porous | 188.6 | 315.5 | 188.6 | 188.6 | ---- |
| MCC | ---- | ---- | ---- | ---- | ---- |
| Lactose 200 m | ---- | ---- | ---- | ---- | 126.9 |
| Primojel | ---- | 20 | 20 | 20 | 20 |
| Mg stearate | 2.5 | 2.5 | 2.5 | 2.5 | 2.5 |
| Compaction process | | | | | |
| HFS (rpm) | 37 | 50 | 50 | 50 | 30 |
| VFS (rpm) | 225 | 225 | 225 | 225 | 225 |
| N1 (rpm) | 3 | 3 | 3 | 3 | 3 |
| Gap (mm) | 0.6 | 0.6 | 0.5 | 0.7 | 0.7 |
| F (KN/cm) | 5 | 5 | 5 | 5 | 3 |
| Mill process | | | | | |
| N2 (rpm) | 500 | 500 | 500 | 500 | 500 |
| Screen (mm) | 2.5 | 2.5 | 2.5 | 2.5 | 2.5 |
| Powder flow (sec/100 g) | 38 | 36 | 33 | 34 | 40 |
| % < 0.25 mm | 22 | 22 | 19 | 16 | 21 |
| % > 1.0 mm | 37 | 42 | 44 | 48 | 39 |
| d(50%) in microns | 720 | 800 | 820 | 950 | 750 |

**Procedure:**
i) S-Ca, Soda Ash IPH, Magnesium stearate and Sodium starch glycolate (primojel) were sifted through #40 mesh sieve.
ii) S-Ca, Soda Ash IPH (indicated as internal) and a portion (indicated as internal) of Magnesium stearate and optionally the Sodium starch glycolate (Primojel^{®})(indicated as internal) and/or lactose sifted above were mixed to obtain a uniform mixture.
iii) The material of step (ii) was mixed with a roller compactor at the indicated settings
iv) The mixed material was milled and sieved over a screen size of 2.5 mm.
v) The powder flow and particle size distribution were measured.

### 2. Tablet formulation

| Batch tablet | F | | G | | H | | I | | J | |
|---|---|---|---|---|---|---|---|---|---|---|
| Batch granulate | A | | B | | C | | D | | E | |
| Composition tablet (mg/t) | | | | | | | | | | |
| Daglutril calcium (S-Ca) | 622 | | 622 | | 622 | | 622 | | 622 | |
| Soda ash IPH; internal | 188.6 | | 315.5 | | 188.6 | | 188.6 | | ---- | |
| Soda ash IPH; external | ---- | | ---- | | ---- | | 126.9 | | 188.6 | |
| MCC PH200; external | 126.9 | | ---- | | 126.9 | | ---- | | ---- | |
| Lactose 200 m; internal | ---- | | ---- | | ---- | | ---- | | 126.9 | |
| Primojel; internal | ---- | | 20 | | 20 | | 20 | | 20 | |
| Primojel; external | 20 | | ---- | | ---- | | ---- | | ---- | |
| Mg stearate; internal | 2.5 | | 2.5 | | 2.5 | | 2.5 | | 2.5 | |
| Mg stearate; external | 5.0 | | 5.0 | | 5.0 | | 5.0 | | 5.0 | |
| Total non coated tablet mass | 965 | | 965 | | 965 | | 965 | | 965 | |
| Bulk / tapped density (g/ml) | 0.55 / 0.67 | | 0.58 / 0.70 | | ---- | | 0.64 / 0.76 | | 0.61 / 0.74 | |
| Carr-Index (%) | 17.9 | | 17.1 | | ---- | | 15.8 | | 17.6 | |
| Compression force (KN) | 9 | 14 | 12 | 25 | 6 | 15 | 6 | 11 | 5 | 10 |
| Tablet weight | 836 | ---- | 901 | ---- | 883 | ---- | ---- | 977 | ---- | 893 |
| Friability (%) | 0.35 | ---- | 0.76 | ---- | 1.30 | ---- | ---- | 2.33 | ---- | 0.79 |
| Crushing strength (N) | 172 | 200 | 146 | 183 | 128 | 200 | 79 | 147 | 103 | 125 |
| Disintegration time (min) | 56 | ---- | 60 | ---- | 58 | ---- | ---- | 50 | ---- | > 90 |

**Procedure:**
vi) The material of step (iv) was mixed with the remaining quantity of Magnesium stearate (external), Sodium starch glycolate (Primojel^{®})(external), Soda Ash IPH (external) or micro crystalline cellulose (external).
vii) The bulk and tap density was measured and the Carr-Index was calculated.
viii) The material of step (vi) was compressed using a tablet compression machine at the indicated compression force.
ix) The friability, crushing strength, and the disintegration time were measured.

**Example 4. Comparative dissolution study for SLV306 formulation with Soda Ash IPH and a traditionally formulated tablet**

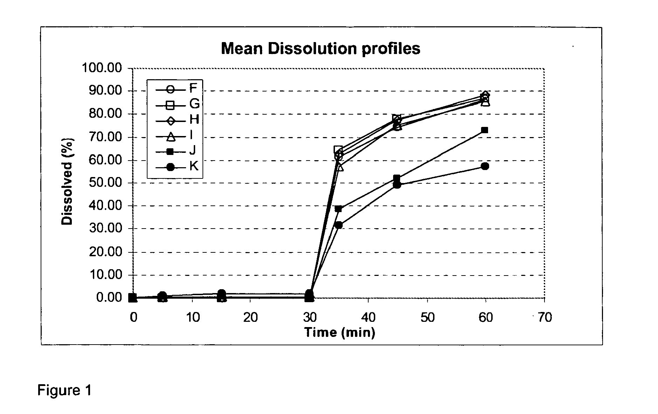

A comparative dissolution study according to the method described in Example 1 (method a) was carried out on one batch of a traditionally formulated tablet (Tablet K, prepared as described in Example 2) and five 600/965 batches of the calcium salt of SLV-306 (S-Ca) prepared according to Example 3 (see batch indications in table)(Tablet F compressed at 9 kN, Tablet G compressed at 12 kN, Tablet H compressed at 6 kN, tablet I compressed at 11 kN and tablet J compressed at 10 kN). The release profile of these formulations is given in the table below and depicted in Figure 1 (○ = Formulation F; □ = Formulation G; ◊ = Formulation H; Δ = Formulation I; ■ = Formulation J; ● = Formulation K)

**Table Release of S-Ca from different formulations**

| Time (minutes) | F (% rel) | G (% rel) | H (% rel) | I (% rel) | J (% rel) | K (% rel) |
|---|---|---|---|---|---|---|
| 0 | 0.00 | 0.00 | 0.01 | 0.13 | 0.00 | 0.0 |
| 5 | 0.04 | 0.19 | 0.19 | 0.41 | 0.25 | 1.1 |
| 15 | 0.13 | 0.21 | 0.22 | 0.40 | 0.23 | 1.6 |
| 30 | 0.14 | 0.12 | 0.25 | 0.32 | 0.26 | 1.9 |
| 35 | 61.10 | 64.45 | 62.54 | 57.35 | 36.74 | 31.4 |
| 45 | 74.44 | 78.15 | 77.61 | 75.02 | 52.26 | 49.1 |
| 60 | 86.43 | 87.03 | 88.40 | 85.75 | 72.91 | 57.4 |

From this study it was concluded that a formulation of S-Ca with a high drug load, having a reasonable size and a favourable release profile was prepared.

## Claims

1. An oral pharmaceutical formulation of an active compound of the general formula 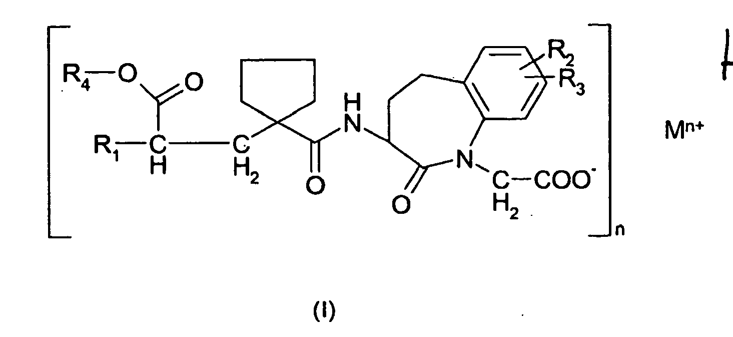 wherein:
R₁ is chosen from:
(1) (C₁-C₆)alkoxy(C₁-C₆)alkyl, which is optionally substituted by a (C₁-C₆)alkoxy;
(2) phenyl-(C₁-C₆)-alkyl and phenyloxy-(C₁-C_{6b}alkyl, wherein the phenyl group is optionally substituted with (C₁-C₆)alkyl, (C₁-C₆)alkoxy or halogen; and
(3) naphtyl-(C₁-C₆)-alkyl;
R₂ and R₃ are both independently hydrogen or halogen,
R₄ is a biolabile ester forming group,
M is a hydrogen or a metal ion, preferably a bivalent metal ion, and nis1,2 or 3;
comprising
a) said active compound in an amount of 10 to 65% of the total weight of the formulation;
b) at least 10% w/w of an alkaline compounds or a mixture of alkaline compounds;
c) optionally comprises auxiliary materials in an amount of between 1% and 45% of the total weight of the formulation
with the proviso that the formulation does not contain a surfactant.

2. A formulation according to claim 1, wherein the alkaline compound is selected from the group consisting of inorganic and organic alkaline compounds, such as sodium bicarbonate, potassium bicarbonate, sodium carbonate, potassium carbonate, sodium citrate, tris buffer, triethanolamine, alkaline hydroxides such as sodium hydroxide, potassium hydroxide or magnesium hydroxide, alkaline phosphates such as dipotassium hydrogen phosphate, and meglumine or mixtures of these alkaline compounds.

3. An improved oral pharmaceutical formulation of an active compound of the general formula 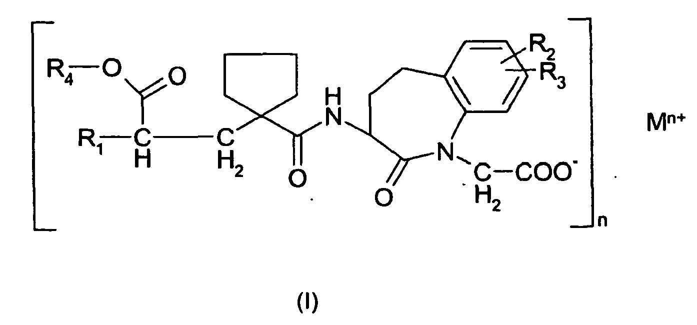 wherein:
R₁ is a selected from the group consisting of (C₁-C₆)alkoxy(C₁-C₆)alkyl which may be substituted by a (C₁-C₆)alkoxy, phenyl-(C₁-C₆)-alkyl and phenyloxy-(C₁-C₆)-alkyl wherein the phenylgroup may be substituted with (C₁-C₆)alkyl,
(C₁-C₆)alkoxy or halogen, and naphtyl-(C₁-C₆)-alkyl,
R₂ and R₃ are both independently hydrogen or halogen,
R₄ is a biolabile ester forming group,
M is a hydrogen or a metal ion, preferably a bivalent metal ion.
nis 1,2 or 3;
comprising
a) said active compound in an amount of 10 to 65% of the total weight of the formulation;
b) at least 10% w/w of a sodium carbonate having a particle size distribution wherein more than 97% of the particles are smaller than 500 µm, more than 40% of the particles are smaller than 160 µm and more than 10% of the particles are smaller than 63 µm;
c) optionally comprises auxiliary materials in an amount of between 1% and 45% of the total weight of the formulation;

4. A formulation according to claim 3, wherein the formulation does not contain a surfactant.

5. A formulation according to claim 3 or 4, wherein said sodium carbonate has a particle size distribution wherein more than 98% of the particles are smaller than 500 µm, more than 60% of the particles are smaller than 160 µm and more than 30% of the particles are smaller than 63 µm.

6. A formulation according to claim 5, wherein said sodium carbonate has a particle size distribution wherein approximately 99.8 % of the particles is smaller than 500 µm, approximately 80% of the particles is smaller than 160 µm and approximately 40% of the particles is smaller than 63 µm.

7. A formulation according to any of claims 3 to 6, wherein said sodium carbonate has a specific surface area of more than 1.0 m²/g.

8. A formulation according to claim 7, wherein said sodium carbonate has a specific surface area of more than 1.5 m²/g.

9. A formulation according to claim 8, wherein said sodium carbonate has a specific surface area of about 2.0 m²/g.

10. A formulation according to any of claims 3 to 9, wherein said sodium carbonate is present in an amount of at least 20% w/w of the formulation.

11. A formulation according to any of claims 1 to 10, wherein M is calcium in its 2+ form.

12. A formulation according to any of claims 1 to 11, **characterised in that** the amount of alkaline compound is more than 55% w/w, preferably more than 60% w/w.

13. A formulation according to any of claims 1 to 12, **characterized in that** said active compound is the calcium salt of 1H-1-Benzazepine-1-acetic acid, 3-[[[1-[2-(ethoxycarbonyl)-4-phenylbutyl]-cyclopentyl]carbonyl]-amino]-2,3,4,5-tetrahydro-2-oxo-, preferably in its 3S,2'R form.

14. A formulation according to any of claims 1 to 13 in the form of granules, compressed tablets or capsules.

15. A method of preparing a formulation according to any of claims 1 to 14, comprising the following steps:
a) mixing of the active compound of formula I with an alkaline compound or a mixture of alkaline compounds and optionally with one or more of the auxiliary materials;
b) compacting the mixture
c) milling and sieving of the granules obtained from said compacting and optionally mixing said sieved granules with one or more auxiliary materials; and
d) optionally compressing of the mixture into tablets, optionally followed by coating, and/or optionally filling the mixture into capsules.

16. A method of preparing a formulation according to any of claims 1 to 14, comprising the following steps:
a) mixing of the active compound with the one or more auxiliary materials;
b) granulating said mixture with an organic solvent;
c) removing the organic solvent to obtain granules;
d) milling and sieving the granules and mixing of the sieved granules with the remaining portion of the auxiliary materials;
e) optionally compressing the mixture into tablets, optionally followed by coating, and/or optionally filling the mixture into capsules.

17. A pharmaceutical formulation according to any of claims 1 to 14 wherein the formulation has a dissolution of at least 50% within 5 minutes as measured using the USP apparatus 2 configuration at a paddle speed of 50 rpm at 37.0 °C and at pH 6.8.

18. A pharmaceutical formulation according to claim 17, wherein the formulation has a dissolution of at least 65% within 15 minutes as measured using the USP apparatus 2 configuration at a paddle speed of 50 rpm at 37.0 °C and at pH 6.8.

19. An oral pharmaceutical formulation according to claim 17, wherein the formulation has a dissolution of at least 75% within 30 minutes as measured using the USP apparatus 2 configuration at a paddle speed of 50 rpm at 37.0 °C and at pH 6.8.

20. An oral pharmaceutical formulation according to claim 17, wherein the formulation has a dissolution of at least 50% within 5 minutes, 65% within 15 minutes and 75% within 30 minutes as measured using the USP apparatus 2 configuration at a paddle speed of 50 rpm at 37.0 °C and at pH 6.8.

21. An oral pharmaceutical formulation according to any of claims 17 to 20 wherein the formulation has a dissolution of less than 5% within 30 minutes as measured using the USP apparatus 2 configuration at a paddle speed of 50 rpm af 37.0 °C and at pH 2.0.

## Patentansprüche

1. Orale pharmazeutische Formulierung einer aktiven Verbindung der allgemeinen Formel 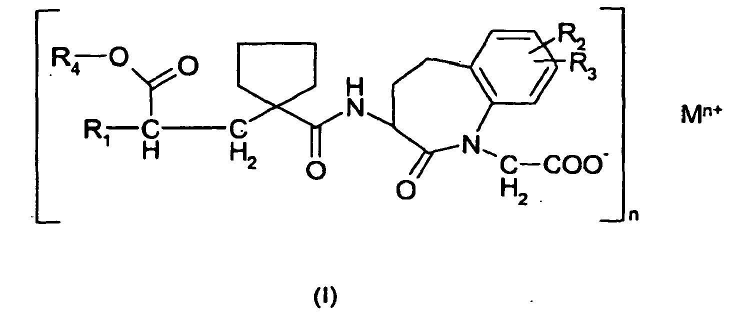 worin:
R₁ ausgewählt ist aus:
(1) (C₁-C₆)Alkoxy(C₁-C₆)alkyl, welches gegebenenfalls substituiert ist durch ein (C₁-C₆)Alkoxy;
(2) Phenyl-(C₁-C₆)-alkyl und Phenyloxy-(C₁-C₆)-alkyl, worin die Phenylgruppe gegebenenfalls substituiert ist mit (C₁-C₆)Alkyl, (C₁-C₆)Alkoxy oder Halogen; und
(3) Naphtyl- (C₁-C₆)-alkyl;
R₂ und R₃ beide unabhängig Wasserstoff oder Halogen darstellen,
R₄ eine einen biolabilen Ester bildende Gruppe darstellt,
M ein Wasserstoff- oder ein Metallion, vorzugsweise ein zweiwertiges Metallion darstellt, und
n 1, 2 oder 3 ist;
welche umfasst:
a) besagte aktive Verbindung in einer Menge von 10 bis 65% des Gesamtgewichts der Formulierung;
b) mindestens 10% Gew./Gew. einer alkalischen Verbindung oder eines Gemisches aus alkalischen Verbindungen;
c) gegebenenfalls Hilfsstoffe in einer Menge zwischen 1% und 45% des Gesamtgewichts der Formulierung umfasst mit der Maßgabe, dass die Formulierung keinen Surfactant enthält.

2. Formulierung gemäß Anspruch 1, worin die alkalische Verbindung ausgewählt ist aus der Gruppe bestehend aus anorganischen und organischen alkalischen Verbindungen wie Natriumbicarbonat, Kaliumbicarbonat, Natriumcarbonat, Kaliumcarbonat, Natriumcitrat, Tris-Puffer, Triethanolamin, alkalischen Hydroxiden wie Natriumhydroxid, Kaliumhydroxid oder Magnesiumhydroxid, alkalischen Phosphaten wie Dikaliumhydrogenphosphat, und Meglumin oder Gemischen aus diesen alkalischen Verbindungen.

3. Verbesserte orale pharmazeutische Formulierung einer aktiven Verbindung der allgemeinen Formel 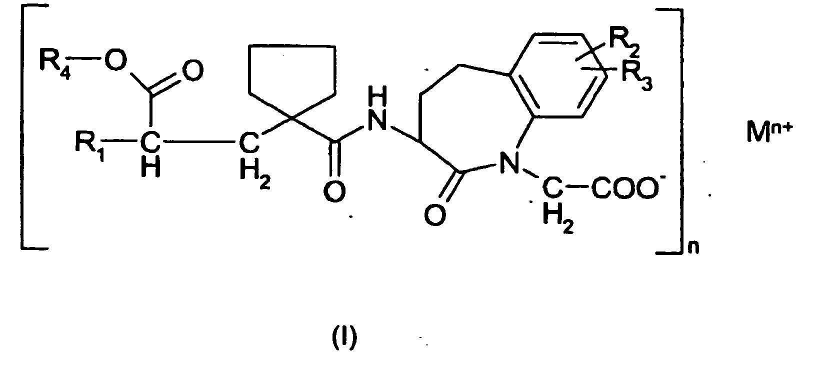 worin:
R₁ ausgewählt ist aus der Gruppe bestehend aus (C₁-C₆)Alkoxy(C₁-C₆)alkyl, welches substituiert sein kann durch ein (C₁-C₆)Alkoxy, Phenyl-(C₁-C₆)-alkyl und Phenyloxy-(C₁-C₆)-alkyl, worin die Phenylgruppe substituiert sein kann mit (C₁-C₆)Alkyl, (C₁-C₆)Alkoxy oder Halogen, und Naphtyl-(C₁-C₆)-alkyl,
R₂ und R₃ beide unabhängig Wasserstoff oder Halogen darstellen,
R₄ eine einen biolabilen Ester bildende Gruppe darstellt,
M ein Wasserstoff- oder ein Metallion, vorzugsweise ein zweiwertiges Metallion darstellt,
n 1, 2 oder 3 ist;
welche umfasst:
a) besagte aktive Verbindung in einer Menge von 10 bis 65% des Gesamtgewichts der Formulierung;
b) mindestens 10% Gew./Gew. eines Natriumcarbonats mit einer Partikelgrößenverteilung, worin mehr als 97% der Partikel kleiner als 500 µm sind, mehr als 40% der Partikel kleiner als 160 µm sind und mehr als 10% der Partikel kleiner als 63 µm sind;
c) gegebenenfalls Hilfsstoffe in einer Menge zwischen 1% und 45% des Gesamtgewichts der Formulierung umfasst.

4. Formulierung gemäß Anspruch 3, wobei die Formulierung keinen Surfactant enthält.

5. Formulierung gemäß Anspruch 3 oder 4, worin besagtes Natriumcarbonat eine Partikelgrößenverteilung hat, worin mehr als 98% der Partikel kleiner als 500 µm sind, mehr als 60% der Partikel kleiner als 160 µm sind und mehr als 30% der Partikel kleiner als 63 µm sind.

6. Formulierung gemäß Anspruch 5, worin besagtes Natriumcarbonat eine Partikelgrößenverteilung hat, worin annähernd 99,8% der Partikel kleiner als 500 µm sind, annähernd 80% der Partikel kleiner als 160 µm sind und annähernd 40% der Partikel kleiner als 63 µm sind.

7. Formulierung gemäß einem von Ansprüchen 3 bis 6, worin besagtes Natriumcarbonat eine spezifische Oberfläche von mehr als 1,0 m²/g hat.

8. Formulierung gemäß Anspruch 7, worin besagtes Natriumcarbonat eine spezifische Oberfläche von mehr als 1,5 m²/g hat.

9. Formulierung gemäß Anspruch 8, worin besagtes Natriumcarbonat eine spezifische Oberfläche von etwa 2,0 m²/g hat.

10. Formulierung gemäß einem von Ansprüchen 3 bis 9, worin besagtes Natriumcarbonat in einer Menge von mindestens 20% Gew./Gew. der Formulierung vorhanden ist.

11. Formulierung gemäß einem von Ansprüchen 1 bis 10, worin M Calcium in seiner 2+ Form darstellt.

12. Formulierung gemäß einem von Ansprüchen 1 bis 11, **dadurch gekennzeichnet, dass** die Menge an alkalischer Verbindung mehr als 55% Gew./Gew., vorzugsweise mehr als 60% Gew./Gew. ist.

13. Formulierung gemäß einem von Ansprüchen 1 bis 12, **dadurch gekennzeichnet, dass** besagte aktive Verbindung das Calciumsalz von 1H-1-Benzazepin-1-essigsäure, 3-[[[1-[2-(Ethoxycarbonyl)-4-phenylbutyl]-cyclopentyl]carbonyl]-amino]-2,3,4,5-tetrahydro-2-oxo-, vorzugsweise in seiner 35,2'R-Form, ist.

14. Formulierung gemäß einem von Ansprüchen 1 bis 13 in der Form von Granalien, komprimierten Tabletten oder Kapseln.

15. Verfahren zum Herstellen einer Formulierung gemäß einem von Ansprüchen 1 bis 14, welches die folgenden Schritte umfasst:
a) Mischen der aktiven Verbindung der Formel I mit einer alkalischen Verbindung oder einem Gemisch aus alkalischen Verbindungen und gegebenenfalls mit einem oder mehreren der Hilfsstoffe;
b) Kompaktieren des Gemisches
c) Mahlen und Sieben der von besagtem Kompaktieren erhaltenen Granalien und gegebenenfalls Mischen der besagten gesiebten Granalien mit einem oder mehreren Hilfsstoffen; und
d) gegebenenfalls Komprimieren des Gemisches in Tabletten, gegebenenfalls gefolgt von Überziehen, und/oder gegebenenfalls Füllen des Gemisches in Kapseln.

16. Verfahren zum Herstellen einer Formulierung gemäß einem von Ansprüchen 1 bis 14, welches die folgenden Schritte umfasst:
a) Mischen der aktiven Verbindung mit dem einem oder mehreren Hilfsstoffen;
b) Granulieren des besagten Gemisches mit einem organischen Lösungsmittel;
c) Entfernen des organischen Lösungsmittels, um Granalien zu erhalten;
d) Mahlen und Sieben der Granalien und Mischen der gesiebten Granalien mit dem verbleibenden Anteil der Hilfsstoffe;
e) gegebenenfalls Komprimieren des Gemisches in Tabletten, gegebenenfalls gefolgt von Überziehen, und/oder gegebenenfalls Füllen des Gemisches in Kapseln.

17. Pharmazeutische Formulierung gemäß einem von Ansprüchen 1 bis 14, wobei die Formulierung eine Auflösung von mindestens 50% innerhalb von 5 Minuten hat, wie unter Verwendung der USP-Apparat 2 Konfiguration bei einer Paddelgeschwindigkeit von 50 U/Min. bei 37,0°C und bei pH 6,8 gemessen.

18. Pharmazeutische Formulierung gemäß Anspruch 17, wobei die Formulierung eine Auflösung von mindestens 65% innerhalb von 15 Minuten hat, wie unter Verwendung der USP-Apparat 2 Konfiguration bei einer Paddelgeschwindigkeit von 50 U/Min. bei 37,0°C und bei pH 6,8 gemessen.

19. Orale pharmazeutische Formulierung gemäß Anspruch 17, wobei die Formulierung eine Auflösung von mindestens 75% innerhalb von 30 Minuten hat, wie unter Verwendung der USP-Apparat 2 Konfiguration bei einer Paddelgeschwindigkeit von 50 U/Min. bei 37,0°C und bei pH 6,8 gemessen.

20. Orale pharmazeutische Formulierung gemäß Anspruch 17, wobei die Formulierung eine Auflösung von mindestens 50% innerhalb von 5 Minuten, 65% innerhalb von 15 Minuten und 75% innerhalb von 30 Minuten hat, wie unter Verwendung der USP-Apparat 2 Konfiguration bei einer Paddelgeschwindigkeit von 50 U/Min. bei 37,0°C und bei pH 6,8 gemessen.

21. Orale pharmazeutische Formulierung gemäß einem von Ansprüchen 17 bis 20, wobei die Formulierung eine Auflösung von weniger als 5% innerhalb von 30 Minuten hat, wie unter Verwendung der USP-Apparat 2 Konfiguration bei einer Paddelgeschwindigkeit von 50 U/Min. bei 37,0°C und bei pH 2,0 gemessen.

## Revendications

1. Formulation pharmaceutique orale d'un composé actif de la
formule générale 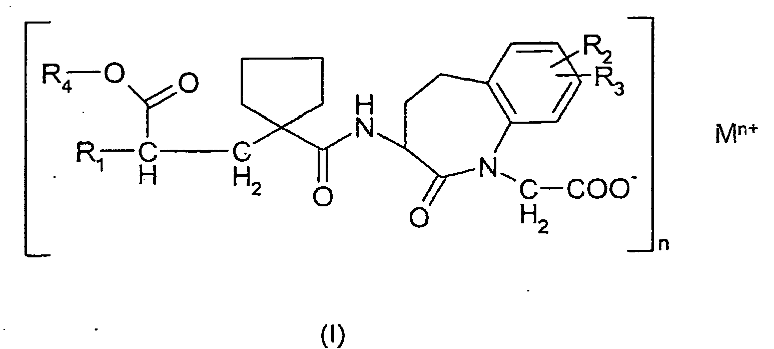 dans laquelle
R₁ est choisi parmi :
(1) un groupe (alcoxy en C₁-C₆)-(alkyle en C₁-C₆) qui est éventuellement substitué par un groupe alcoxy en C₁-C₆ ;
(2) un groupe phényle-(alkyle en C₁-C₆) et phényloxy-(alkyle en C₁-C₆), dans lequel le groupe phényle est éventuellement substitué par un groupe alkyle en C₁-C₆, alcoxy en C₁-C₆ ou un atome d'halogène ; et
(3) un groupe naphtyle-(alkyle en C₁-C₆) ;
R₂ et R₃ sont tous deux indépendamment un atome d'hydrogène ou un atome d'halogène,
R₄ est un groupe formant un ester biolabile,
M est un atome d'hydrogène ou un ion de métal, de préférence un ion de métal bivalent, et
n est égal à 1, 2 ou 3 ;
comprenant
a) ledit composé actif dans une quantité de 10 à 65 % de la masse totale de la formulation ;
b) au moins 10 % en masse/masse d'un composé alcalin ou d'un mélange de composés alcalins ;
c) comprend éventuellement des matériaux auxiliaires dans une quantité comprise entre 1 % et 45 % de la masse totale de la formulation à la condition que la formulation ne contienne pas de tensioactif.

2. Formulation selon la revendication 1, dans laquelle le composé alcalin est choisi dans le groupe constitué de composés alcalins inorganiques et organiques, tels que le bicarbonate de sodium, le bicarbonate de potassium, le carbonate de sodium, le carbonate de potassium, le citrate de sodium, un tampon tris, la triéthanolamine, des hydroxydes alcalins, tels que l'hydroxyde de sodium, l'hydroxyde de potassium ou l'hydroxyde de magnésium, des phosphates alcalins, tels que l'hydrogénophosphate de dipotassium et le méglumine ou des mélanges de ces composés alcalins.

3. Formulation pharmaceutique orale améliorée d'un composé actif de la formule générale 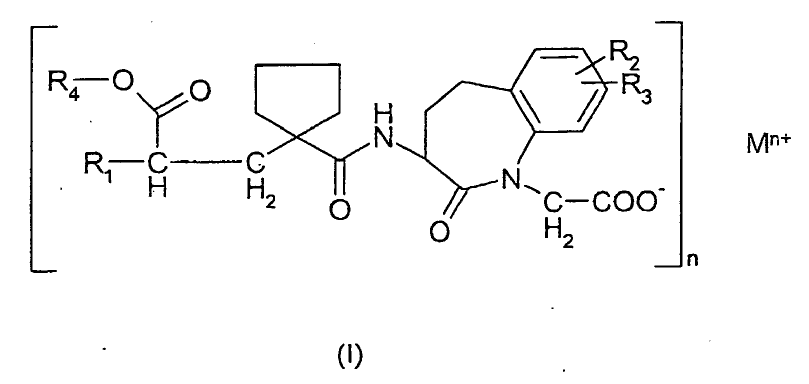 dans laquelle
R₁ est choisi parmi dans le groupe constitué d'un groupe (alcoxy en C₁-C₆)-(alkyle en C₁-C₆) qui peut être substitué par un groupe alcoxy en C₁-C₆; d'un groupe phényle-(alkyle en C₁-C₆) et phényloxy-(alkyle en C₁-C₆), dans lequel le groupe phényle peut être substitué par un groupe alkyle en C₁-C₆, alcoxy en C₁-C₆ ou un atome d'halogène ; et d'un groupe naphtyle-(alkyle en C₁-C₆) ;
R₂ et R₃ sont tous deux indépendamment un atome d'hydrogène ou un atome d'halogène,
R₄ est un groupe formant un ester biolabile,
M est un atome d'hydrogène ou un ion de métal, de préférence un ion de métal bivalent, et
n est égal à 1, 2 ou 3 ;
comprenant
a) ledit composé actif dans une quantité de 10 à 65 % de la masse totale de la formulation ;
b) au moins 10 % en masse/masse d'un carbonate de sodium ayant une distribution de taille de particules dans laquelle plus de 97 % des particules sont plus petites que 500 µm, plus de 40 % des particules sont plus petites que 160 µm et plus de 10 % des particules sont plus petites que 63 µm.
c) comprend éventuellement des matériaux auxiliaires dans une quantité comprise entre 1 % et 45 % de la masse totale de la formulation.

4. Formulation selon la revendication 3, dans laquelle la formulation ne contient pas de tensioactif.

5. Formulation selon la revendication 3 ou 4, dans laquelle ledit carbonate de sodium présente une distribution de taille de particules dans laquelle plus de 98 % des particules sont plus petites que 500 µm, plus de 60 % des particules sont plus petites que 160 µm et plus de 30 % des particules sont plus petites que 63 µm.

6. Formulation selon la revendication 5, dans laquelle ledit carbonate de sodium présente une distribution de taille de particules dans laquelle approximativement 99,8 % des particules sont plus petites que 500 µm, approximativement 80 % des particules sont plus petites que 160 µm et approximativement 40 % des particules sont plus petites que 63 µm.

7. Formulation selon l'une quelconque des revendications 3 à 6, dans laquelle ledit carbonate de sodium présente une surface spécifique supérieure à 1,0 m²/g.

8. Formulation selon la revendication 7, dans laquelle ledit carbonate de sodium présente une surface spécifique supérieure à 1,5 m2/g_{.}

9. Formulation selon la revendication 8, dans laquelle ledit carbonate de sodium présente une surface spécifique d'environ 2,0 m²/g.

10. Formulation selon l'une quelconque des revendications 3 à 9, dans laquelle ledit carbonate de sodium est présent dans une quantité d'au moins 20 % en masse/masse de la formulation.

11. Formulation selon l'une quelconque des revendications 1 à 10, dans laquelle M est le calcium dans sa forme 2+.

12. Formulation selon l'une quelconque des revendications 1 à 11, **caractérisée en ce que** la quantité de composé alcalin est supérieure à 55% en masse/masse, de préférence supérieure à 60 % en masse/masse.

13. Formulation selon l'une quelconque des revendications 1 à 12, **caractérisée en ce que** ledit composé actif est le sel de calcium, d'acide 1H-1-benzazépine-1-acétique, 3-[[[1-[2-(éthoxycarbonyl)-4-phénylbutyl]-cyclopentyl]carbonyl]-amino]-2,3,4,5-tétrahydro-2-oxo-, de préférence dans sa forme 3S,2'R.

14. Formulation selon l'une quelconque des revendications 1 à 13 dans la forme de granulés, de comprimés ou de capsules.

15. Procédé de préparation d'une formulation selon l'une quelconque des revendications 1 à 14 comprenant les étapes suivantes consistant :
a) à mélanger le composé actif de la formule I avec un composé alcalin ou un mélange de composés alcalins et éventuellement avec un ou plusieurs des matériaux auxiliaires ;
b) à compacter le mélange
c) à broyer et à tamiser les granulés obtenus à partir dudit compactage et à mélanger éventuellement lesdits granulés tamisés avec un ou plusieurs matériaux auxiliaires ; et
d) à comprimer éventuellement le mélange en comprimés, puis éventuellement à enrober, et/ou éventuellement à remplir des capsules avec le mélange.

16. Procédé de préparation d'une formulation selon l'une quelconque des revendications 1 à 14 comprenant les étapes suivantes consistant :
a) à mélanger le composé actif avec le un ou plusieurs matériaux auxiliaires ;
b) à granuler ledit mélange avec un solvant organique ;
c) à éliminer le solvant organique pour obtenir des granulés ;
d) à broyer et à tamiser les granulés et à mélanger les granulés tamisés avec la partie restante des matériaux auxiliaires ;
e) à comprimer éventuellement le mélange en comprimés, puis éventuellement à enrober, et/ou éventuellement à remplir des capsules avec le mélange.

17. Formulation pharmaceutique selon l'une quelconque des revendications 1 à 14, dans lequel la formulation présente une dissolution d'au moins 50 % en 5 minutes comme mesurée en utilisant la configuration 2 d'appareil USP à une vitesse de pâle de 50 tr/min à 37,0°C et à un pH de 6,8.

18. Formulation pharmaceutique selon la revendication 17, dans laquelle la formulation présente une dissolution d'au moins 65 % en 15 minutes mesurée en utilisant la configuration 2 d'appareil USP à une vitesse de pâle de 50 tr/min à 37,0°C et à un pH de 6,8.

19. Formulation pharmaceutique orale selon la revendication 17, dans laquelle la formulation présente une dissolution d'au moins 75 % en 30 minutes mesurée en utilisant la configuration 2 d'appareil USP à une vitesse de pâle de 50 tr/min à 37,0°C et à un pH de 6,8.

20. Formulation pharmaceutique orale selon la revendication 17, dans laquelle la formulation présente une dissolution d'au moins 50 % en 5 minutes, 65 % en 15 minutes et 75 % en 30 minutes mesurée en utilisant la configuration 2 d'appareil USP à une vitesse de pâle de 50 tr/min à 37,0°C et à un pH de 6,8.

21. Formulation pharmaceutique orale selon l'une quelconque des revendications 17 à 20, dans laquelle la formulation présente une dissolution inférieure à 5 % en 30 minutes mesurée en utilisant la configuration 2 d'appareil USP à une vitesse de pâle de 50 tr/min à 37,0°C et à un pH de 2,0.
